# EUROPÄISCHE PATENTANMELDUNG

(11) **EP 4 324 905 A1**
(43) Veröffentlichungstag der Anmeldung: **21.02.2024**
(21) Anmeldenummer: 22191238.9
(22) Anmeldetag: 19.08.2022
(51) Int. Cl.: C12M 1/00, C12M 1/34

(54) **BIOREAKTORSYSTEM MIT BEGASUNGSANLAGE**

(71) Anmelder: Sartorius Stedim Biotech GmbH, 37079 Göttingen (DE)
(72) Erfinder: Leupold, Marco, 34302 Guxhagen (DE); Husemann, Bernward, 34302 Guxhagen (DE); Steinicke, Robert, 37079 Göttingen (DE); Becker, Sören, 37079 Göttingen (DE); Adams, Thorsten, 37079 Göttingen (DE)
(74) Vertreter: Prinz & Partner mbB

(57) **Zusammenfassung**

Ein Bioreaktorsystem (10) zur Durchführung eines biologischen Prozesses umfasst einen Behälter (12) zur Aufnahme eines flüssigen biologischen Mediums und eine Begasungsanlage zur kontrollierten Zufuhr verschiedener Gase von Gasquellen (38, 40, 42, 44) in den Behälter (12). Die Begasungsanlage weist mehrere Gasauslassleitungen (28, 30, 32) auf, die in eine oder mehrere Begasungsvorrichtungen (20) und/oder in einen Overlay-Gasauslass im Inneren des Behälters (12) münden. Jede Gasauslassleitung (28, 30, 32) ist über jeweils eine Gaszufuhrleitung (46, 52, 56, 58) oder jeweils einen Abzweig (48, 50, 54, 60) von einer Gaszufuhrleitung (46, 52, 56, 58) mit mehreren Gasquellen (38, 40, 42, 44) verbunden. In jeder Gaszufuhrleitung (46, 52, 56, 58) und in jedem Abzweig (48, 50, 54, 60) ist ein mit einer Steuereinheit verbundener Massendurchflussregler (62) angeordnet. Ein solches Bioreaktorsystem zur Durchführung eines biologischen Prozesses umfasst vorzugsweise eine Steuerung, die einen Führungsregler für eine Regelgröße, wenigstens einen dem Führungsregler zugeordneten Sensor und einen oder mehrere Folgeregler mit Stellgliedern (14, 62) umfasst, deren Stellgrößen die Regelgröße gezielt beeinflussen. Die Steuerung ist so eingerichtet, dass dem Führungsregler ein Sollwert für die Regelgröße vorgegeben werden kann, der wenigstens eine Sensor zur wiederholten Bestimmung eines Istwerts der Regelgröße verwendet wird, und der Führungsregler ein von der Abweichung des Regelgrößen-Istwerts vom Regelgrößen-Sollwert abhängiges Ausgangssignal auf die Folgeregler gibt. Den Folgereglern sind Steuerprofile zugewiesen, die vom Ausgangssignal des Führungsreglers abhängig sind. Gemäß einer ersten Alternative beinhalten die Steuerprofile Sollwerte für die Stellglieder (14, 62) der Folgeregler, und die Steuerung ist so eingerichtet, dass die Sollwerte für die Stellglieder (14, 62) in der physikalischen Einheit von deren Stellgrößen eingegeben werden können. Gemäß einer zweiten Alternative beinhalten die Steuerprofile Sollwerte für einen unmittelbar auf den biologischen Prozess bezogenen, durch die Stellglieder (14, 62) gezielt beeinflussbaren Parameter, und die Steuerung ist so eingerichtet, dass sie den Parameter-Sollwerten automatisch Sollwerte für die Stellglieder der Folgeregler zuweist.

## Beschreibung

Die Erfindung betrifft ein Bioreaktorsystem zur Durchführung eines biologischen Prozesses mit einer Begasungsanlage.

Unter einem Bioreaktorsystem ist hier allgemein eine Apparatur mit einem Behälter zu verstehen, mit der biotechnologische Prozesse, z. B. biopharmazeutische Prozesse, durchgeführt oder unterstützt werden können. Neben Bioreaktoren, in denen Mikroorganismen oder Säugetierzellen unter bestimmten Bedingungen kultiviert werden, sind Prozessmischanlagen, mit denen flüssige biologische Medien wie Suspensionen, Lösungen, Emulsionen oder ähnliches gemischt werden können, ein weiteres Beispiel. Der Behälter des Bioreaktorsystems kann ein formstabiles Gefäß oder ein flexibler Beutel sein.

Ein Bioreaktorsystem kann mit einer oder mehreren Begasungsvorrichtungen (im Englischen "Sparger") ausgestattet sein, um Luft und/oder andere Gase direkt in ein im Behälter vorhandenes flüssiges biologisches Medium einzuleiten. Im biotechnologischen Kontext der Zellkultivierung steuert das Begasen den Luft- bzw. Gaseintrag in eine Nährstoffbrühe im Inneren des Behälters, um eine optimale Umgebung für das Zellwachstum zu schaffen. Daher wird bei Zellkulturen - im Gegensatz zu manchen Bakterienkulturen - nicht nur mit Luft, sondern mit Sauerstoff (O₂), Stickstoff (N₂), Kohlenstoffdioxid (CO₂) und Luft in einer für die jeweilige Zellart und Fermentation optimierten Zusammensetzung begast. Da sich während des Zellwachstums der pH-Wert im Medium ändert, aber für viele Produktionsprozesse ein konstanter pH-Wert essentiell ist, muss dieser auch geregelt werden. Die Regelung wird zumeist über kontrollierte Begasung mit CO₂ durchgeführt.

Als Begasungsvorrichtungen kommen typischerweise Begasungsringe oder -scheiben zum Einsatz, oder ein Gas wird direkt über eine Gasauslassleitung in das flüssige biologische Medium eingebracht. Es gibt auch kombinierte Begasungsvorrichtungen, die ein Gehäuse mit zwei Begasungskanälen im Inneren aufweisen. Über zwei Gaseinlassöffnungen im Gehäuse können jeweils ein bzw. mehrere Gase in einen zugeordneten Begasungskanal des Gehäuses eingeleitet werden. Dem ersten Begasungskanal ist eine Vielzahl von größeren Gasauslassöffnungen mit einem Durchmesser von mehr als 0,2 mm, z. B. 0,8 mm, zugeordnet, während dem zweiten Begasungskanal eine Vielzahl von kleineren Gasauslassöffnungen mit einem kleineren Durchmesser, z. B. 0,15mm oder kleiner zugeordnet ist. Die kombinierte Begasungsvorrichtung kann somit wahlweise als sog. "Ringsparger" unter Ausnutzung der größeren Öffnungen und als "Microsparger" unter Ausnutzung der kleineren Öffnungen eingesetzt werden.

Zusätzlich kann ein Gas von oben in den Bioreaktorbehälter zugeführt werden, genauer gesagt in den Raum oberhalb des Mediums. Eine solche sog. "Overlay"- oder Kopfbegasung kann aus verschiedenen Gründen erfolgen, u. a. um bei Verwendung eines Bioreaktorsystems mit einem flexiblen Beutel sicherzustellen, dass der Beutel aufgeblasen bleibt.

Für die Zufuhr verschiedener Gase in den Bioreaktorbehälter ist üblicherweise eine Gaszufuhrstation mit Gasleitungen vorgesehen, die von den entsprechenden Gasquellen zu den Gasauslässen im Behälter führen, d.h. - sofern vorgesehen - zum Ringsparger und Microsparger sowie zu einem Overlay-Gasauslass, wobei sich Ringsparger und Microsparger eine Gasleitung teilen. Zur Steuerung der Gasmengenzufuhr können Massendurchflussregler und zur Schaltung der Strömungswege (von der jeweiligen Gasquelle zum gewünschten Gasauslass) Sperrventile verwendet werden. Bei einer solchen konventionellen Konfiguration muss für ein bestimmtes Gas vorher entschieden werden, ob es über den Ringsparger, über den Microsparger oder zum Overlay-Gasauslass zugeführt wird. Der jeweilige Strömungsweg wird durch entsprechendes Schalten der Sperrventile freigegeben. Dadurch sind jedoch die Möglichkeiten der Begasung limitiert.

Aufgabe der Erfindung ist es, eine flexiblere Zufuhr von Gas in einen Bioreaktorbehälter zu ermöglichen.

Gelöst wird diese Aufgabe durch ein Bioreaktorsystem mit den Merkmalen des Anspruchs 1. Vorteilhafte und zweckmäßige Ausgestaltungen des erfindungsgemäßen Bioreaktorsystems sind in den Unteransprüchen angegeben.

Das erfindungsgemäße Bioreaktorsystem zur Durchführung eines biologischen Prozesses umfasst einen Behälter zur Aufnahme eines flüssigen biologischen Mediums und eine Begasungsanlage zur kontrollierten Zufuhr verschiedener Gase von Gasquellen in den Behälter. Die Begasungsanlage weist mehrere Gasauslassleitungen auf, die in eine oder mehrere Begasungsvorrichtungen und/oder in einen Overlay-Gasauslass im Inneren des Behälters münden. Jede Gasauslassleitung ist über jeweils eine Gaszufuhrleitung oder jeweils einen Abzweig von einer Gaszufuhrleitung mit mehreren Gasquellen verbunden. In jeder Gaszufuhrleitung und in jedem Abzweig ist ein mit einer Steuereinheit verbundener Massendurchflussregler angeordnet.

Die Konfiguration der Begasungsanlage des erfindungsgemäßen Bioreaktorsystems sieht demnach vor, dass jede Verbindung zwischen einer Gasquelle und einer Gasauslassleitung, über die Gas in den Bioreaktorbehälter zugeführt wird, mit einem eigenen Massendurchflussregler versehen ist. Die Gasauslassleitungen der Begasungsanlage sind typischerweise mit Begasungsvorrichtungen in Form von Spargern, insbesondere einem Ringsparger und einem Microsparger oder einem kombinierten Sparger, und/oder mit einem Overlay-Gasauslass verbunden. Somit kann jeder Sparger und jeder Overlay-Gasauslass individuell mit Gas versorgt werden. Das Vorsehen eigener Massendurchflussregler in allen Verbindungen ermöglicht es auch, eine Gasart (z.B. Sauerstoff) gleichzeitig und individuell geregelt über mehrere Sparger, insbesondere einen Ringsparger und einen Microsparger und ggf. weitere Sparger (anderer Typen), und/oder weitere Gasauslassleitungen in den Bioreaktorbehälter zu leiten. Auch ein Mischen von verschiedenen Gasen mit variablen Anteilen ist möglich.

Weitere Vorteile einer solchen Bioreaktor-Konfiguration sind eine genauere und feinere Regelung der Gaszufuhr, die eine gezielte Einstellung der Blasengröße und der Blasenanzahl erlaubt, und eine Gasersparnis.

Bei einer bevorzugten Ausführungsform des erfindungsgemäßen Bioreaktorsystems ist eine erste Gasauslassleitung, die in einen Ringsparger mündet, mit einer Luftquelle und/oder einer Sauerstoffquelle und/oder einer Stickstoffquelle und/oder einer Kohlendioxidquelle verbunden. Eine zweite Gasauslassleitung, die in einen Microsparger mündet, ist vorzugsweise mit einer Luftquelle und/oder einer Sauerstoffquelle verbunden. Eine dritte Gasauslassleitung, die in einen Overlay-Gasauslass mündet, ist vorzugsweise mit einer Luftquelle und/oder einer Kohlendioxidquelle verbunden.

Gemäß einem eigenständigen Erfindungsaspekt umfasst ein Bioreaktorsystem zur Durchführung eines biologischen Prozesses, insbesondere ein Bioreaktorsystem der oben beschriebenen Art, eine Steuerung, die einen Führungsregler für eine Regelgröße, wenigstens einen dem Führungsregler zugeordneten Sensor und einen oder mehrere Folgeregler mit Stellgliedern umfasst, deren Stellgrößen die Regelgröße gezielt beeinflussen. Die Steuerung ist so eingerichtet ist, dass dem Führungsregler ein Sollwert für die Regelgröße vorgegeben werden kann, der wenigstens eine Sensor zur wiederholten Bestimmung eines Istwerts der Regelgröße verwendet wird, und der Führungsregler ein von der Abweichung des Regelgrößen-Istwerts vom Regelgrößen-Sollwert abhängiges Ausgangssignal auf die Folgeregler gibt. Den Folgereglern sind Steuerprofile zugewiesen, die vom Ausgangssignal des Führungsreglers abhängig sind. Gemäß einer ersten Alternative beinhalten die Steuerprofile Sollwerte für die Stellglieder der Folgeregler, und die Steuerung ist so eingerichtet, dass die Sollwerte für die Stellglieder in der physikalischen Einheit von deren Stellgrößen eingegeben werden können. Gemäß einer zweiten Alternative beinhalten die Steuerprofile Sollwerte für einen unmittelbar auf den biologischen Prozess bezogenen, durch die Stellglieder gezielt beeinflussbaren Parameter, und die Steuerung ist so eingerichtet, dass sie den Parameter-Sollwerten automatisch Sollwerte für die Stellglieder der Folgeregler zuweist.

Dank der erfindungsgemäß eingerichteten Steuerung muss der Anwender nicht in generischen Einheiten wie Prozentwerten von Ausgabesignalen von Folgereglern denken und entsprechend abstrakte Daten eingeben.

Vielmehr kann der Anwender gemäß der ersten Alternative der Erfindung für die Sollwerte der Stellglieder, wie etwa einen im Bioreaktorbehälter angeordneten Rührer und Massendurchflussregler einer Begasungsanlage, echte physikalische Werte eingeben, ohne dass er sich überlegen muss, welcher Ausgangswert des Folgereglers einer bestimmten Drehzahl des Rührers oder einer Begasungsrate entspricht.

Gemäß der zweiten Alternative der Erfindung ist die Eingabe des Anwenders noch weniger abstrakt, nämlich derart, dass der Anwender einen Sollwert für einen prozessbezogenen, d.h. den Prozess charakterisierenden Parameter vorgibt, z.B. einen gewünschten Wert für den volumenbezogenen Stoffübergangskoeffizienten (k_{L}a-Wert). Die Steuerung wendet dann automatisch die diesem Wert zugeordneten Steuerprofile auf die Steuerung der Stellglieder der Folgeregler an, insbesondere den Rührer und die Massendurchflussregler. Dies ist insbesondere dann von Vorteil, wenn der Anwender einen Bioprozess im kleinen Labormaßstab entwickelt hat und nicht weiß, wie er beim Skalieren auf einen großen Produktionsmaßstab die Stellglieder eines großen Bioreaktorsystems einstellen soll, um den gleichen Parameterwert zu erreichen und zu halten. Die auf das große Bioreaktorsystem abgestimmte Steuerung erledigt dies automatisch; der Anwender muss nur den gewünschten Parameterwert eingeben.

Abgesehen von diesen Vorzügen trägt die Erfindung ganz erheblich dazu bei, das Risiko von Fehleingaben zu reduzieren.

Als prozessbezogener Parameterwert, den der Anwender anstelle von Prozentwerten für die Folgeregler eingibt, kommt insbesondere ein Wert für einen der folgenden Parameter in Betracht: Zellwachstumsrate, Zelldichte, Sauerstofftransferrate, Sauerstoffaufnahmerate, Blasengröße, Blasenanzahl, Mischzeit, volumenbezogener Stoffübergangskoeffizient.

Diese Parameter sind aus der Sicht des Anwenders wichtige Parameter, die seinen Prozess charakterisieren. Beispielsweise hat die Blasengröße einen erheblichen Einfluss darauf, wie sich der Sauerstoff im Bioreaktorbehälter verteilt. Bei den von einem Microsparger erzeugten kleinen Blasen ist die Oberflächenspannung die dominierende Kraft. Sie haben daher eine lange Verweildauer im Reaktor, was den Sauerstoff-Massentransfer (Sauerstoffeintrag) verbessert, sie aber für das Strippen von Kohlendioxid aus der Kultur weniger geeignet macht. Die von einem Ringsparger erzeugten größeren Blasen haben eine kürzere Verweilzeit, lösen sich aber weniger leicht auf als kleinere Blasen. Sie dienen insbesondere dem CO₂-Stripping.

Als Regelgröße für den Führungsregler des Bioreaktorsystems eignet sich insbesondere der Gelöstsauerstoff, da ein entsprechender Sensor in der Regel immer vorhanden ist. Die Regelgröße kann grundsätzlich aber auch ein anderer Parameter sein, der direkt mit dem wenigstens einen Sensor messbar ist, wie etwa einer der Folgenden: Kohlenstoffdioxidgehalt, Zellzahl, Zelldichte, Lebendzellvolumen, Trübung, Glukosegehalt, pH-Wert, Kohlenstoffdioxidpartialdruck, Sauerstoffpartialdruck.

Gemäß einer Weiterbildung der Erfindung kann die Regelgröße sogar ein Parameter sein, der nicht direkt mit dem wenigstens einen Sensor messbar, aber mithilfe des wenigstens einen Sensors bestimmbar ist, wie etwa einer der Folgenden: Sauerstofftransferrate, Sauerstoffaufnahmerate, Zellwachstumsrate, Glukoseverbrauchsrate, Kohlenstoffdioxidbildungsrate, Kohlenstoffdioxidaustragungsrate, respiratorischer Quotient, Biomasseertrag, Glukoseausbeute, Ausbeute einer anderen Kohlenstoff- oder Stickstoffquelle. Zur Bestimmung des Parameter-Istwerts werden zusätzlich Berechnungen vorgenommen und/oder ein zuvor erstelltes mathematisches Modell angewendet. Der Sensor und die notwendigen Hilfsmittel können in einem solchen Fall gemeinsam als "Softsensor" bezeichnet werden.

Bei einer bevorzugten Ausführungsform des erfindungsgemäßen Bioreaktorsystems umfassen die Stellglieder einen in einem Behälter des Bioreaktorsystems angeordneten Rührer und mehrere Massendurchflussregler einer Begasungsanlage. Die Steuerung kann dann gemäß der ersten Alternative der Erfindung so eingerichtet sein, dass als Sollwerte für den Rührer Werte in der Einheit U/min (Umdrehungen pro Minute) und als Sollwerte für die Massendurchflussregler Werte in der Einheit L/min (Liter pro Minute) eingegeben werden können. Dies soll natürlich davon abgeleitete oder ableitbare Einheiten wie etwa U/sec (Umdrehungen pro Sekunde) bzw. mUsec (Milliliter pro Sekunde) etc. einschließen, da es sich immer noch um physikalische Einheiten der Stellgröße des Rührers bzw. der Massendurchflussregler handelt - im Gegensatz zu Prozentwerten bezogen auf den Regelbereich des jeweiligen Folgereglers.

Die den Folgereglern zugewiesenen, vom Ausgangssignal des Führungsreglers abhängigen Steuerprofile können direkt in der Steuerung oder in einer externen Datenbank hinterlegt sein, auf die die Steuerung Zugriff hat. Die Steuerprofile selbst können vom Hersteller oder vom Anwender vorgegeben sein, oder sie können vom Anwender individuell voreingestellt werden.

Vorzugsweise basieren die Steuerprofile auf empirisch ermittelten Werten oder auf einem zuvor erstellten mathematischen Modell.

Eine Weiterbildung der Erfindung sieht vor, dass die Steuerung die Eingaben der Sollwerte für die Stellglieder der Folgeregler, insbesondere den Rührer und die Massendurchflussregler, dahingehend überwacht, dass keine Sollwertkombinationen eingegeben werden, die zu einem nicht-ordnungsgemäßen Betrieb des Bioreaktorsystems führen würden. Dadurch können insbesondere ein Überflutungsverhalten oder unnötig hohe Leistungseinträge ohne Begasung vermieden werden.

Zudem können mit der Steuerung die zeitliche Abfolge (Startzeitpunkt) und die Ansteuerung der jeweiligen Stellglieder automatisch vorgegeben werden. Beispielsweise können höhere k_{L}a-Werte und eine gleichmäßigere Blasenverteilung erreicht werden, indem zuerst die Begasung und im Anschluss der Rührer gestartet wird.

Weitere Merkmale und Vorteile der Erfindung ergeben sich aus der nachfolgenden Beschreibung und aus den beigefügten Zeichnungen, auf die Bezug genommen wird. In den Zeichnungen zeigen:
- Figur 1 ein Bioreaktorsystem mit einer Begasungsanlage;
- Figur 2 eine stark vereinfachte Darstellung einer Begasungsvorrichtung;
- Figur 3 ein Schaltbild einer herkömmlichen Gaszufuhrsteuerung eines Bioreaktorsystems;
- Figur 4 ein Schaltbild einer Gaszufuhrsteuerung eines erfindungsgemäßen Bioreaktorsystems;
- Figur 5 mehrere auf eine Regelgröße abgestimmte Steuerprofile zur Ansteuerung verschiedener Stellglieder während eines Bioprozesses.

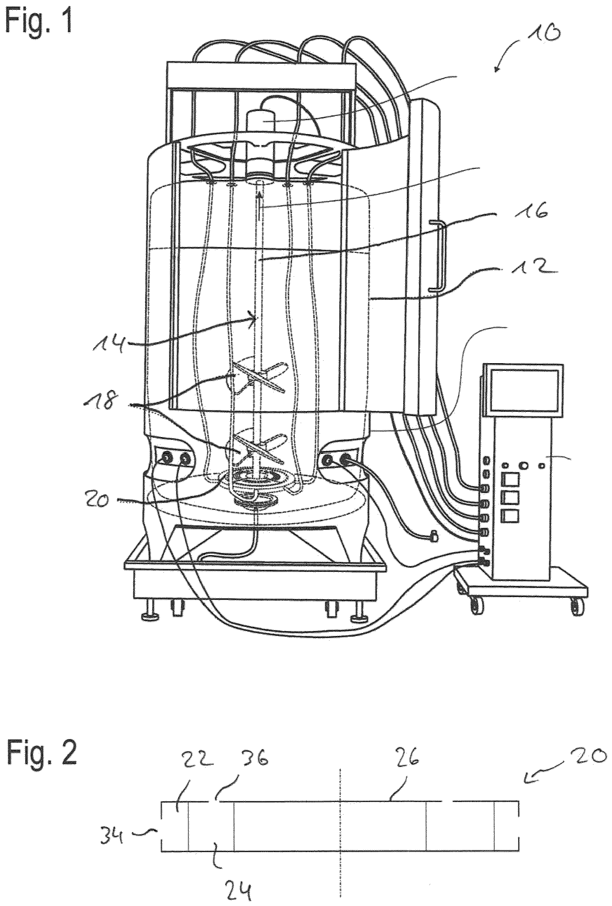

In Figur 1 ist eine beispielhafte Ausführungsform eines Bioreaktorsystems 10 zur Kultivierung von Zellen dargestellt, der einen Behälter 12 umfasst, hier in Form eines flexiblen Einwegbeutels. Der Behälter 12 ist zur Aufnahme eines im Wesentlichen flüssigen biologischen Mediums vorgesehen, das als Nährmedium dient.

Das Bioreaktorsystem 10 umfasst des Weiteren einen Rührer 14, der im Inneren des Behälters 12 installiert ist. Der Rührer 14 weist eine Rührerwelle 16 auf, auf der ein oder mehrere Rührorgane 18 angeordnet sind. Der Rührer 14 dient insbesondere zur Durchmischung des biologischen Mediums während der Kultivierung.

Wie ebenfalls in Figur 1 dargestellt, umfasst das Bioreaktorsystem 10 ferner eine im Behälter 12 angeordnete Begasungsvorrichtung 20, die der Zufuhr mehrerer Gase zum biologischen Medium dient, insbesondere Sauerstoff, Kohlenstoffdioxid, Stickstoff und Luft.

Wie in der schematischen Figur 2 zu sehen ist, weist die Begasungsvorrichtung 20 mehrere, hier zwei, voneinander getrennte Begasungskanäle 22, 24 auf, die innerhalb eines Gehäuses 26 gebildet sind. Im Gehäuse 26 ist für jeden Begasungskanal 22, 24 ein Gaseinlass gebildet, über den ein Gas in den jeweiligen Begasungskanal 22, 24 eingeleitet werden kann. Im montierten und betriebsbereiten Zustand steht jeder Gaseinlass über eine entsprechende Gasauslassleitung 28, 30 in Fluidverbindung mit einer entsprechenden Gasquelle.

Ferner weist das Gehäuse 26 eine Mehrzahl von Gasauslassöffnungen 34, 36 auf, über die das Gas aus dem jeweiligen Begasungskanal 22, 24 in das die Begasungsvorrichtung 20 umgebende flüssige biologische Medium im Inneren des Behälters 12 abgeleitet werden kann.

Im dargestellten Ausführungsbeispiel weist der äußere erste Begasungskanal 22 größere Gasauslassöffnungen 34 mit einem Durchmesser von mehr als 0,2 mm, vorzugsweise von 0,25 bis 4,0 mm, bzw. einer Querschnittsfläche von mehr als 0,03 mm², vorzugsweise von 0,05 bis 12,0 mm², auf und entspricht einem Ringsparger. Der innere zweite Begasungskanal 24 weist kleinere Gasauslassöffnungen 36 mit einem Durchmesser von höchstens 0,2 mm, vorzugsweise von 0,005 bis 0,2 mm, bzw. einer Querschnittsfläche von höchstens 0,03 mm², vorzugsweise von 0,00002 bis 0,03 mm², auf und entspricht einem Microsparger. Der Einfachheit halber wird im Folgenden der erste Begasungskanal 22 als Ringsparger 22 und der zweite Begasungskanal 24 als Microsparger 24 bezeichnet.

Zusätzlich zur Begasungsvorrichtung 20 ist eine (in Figur 1 nicht dargestellte) Overlay-Begasung vorgesehen, bei der über eine weitere, in den Raum oberhalb des flüssigen biologischen Mediums in einen Gasauslass mündende Gasauslassleitung 32 Luft oder Kohlenstoffdioxid zugeführt werden kann.

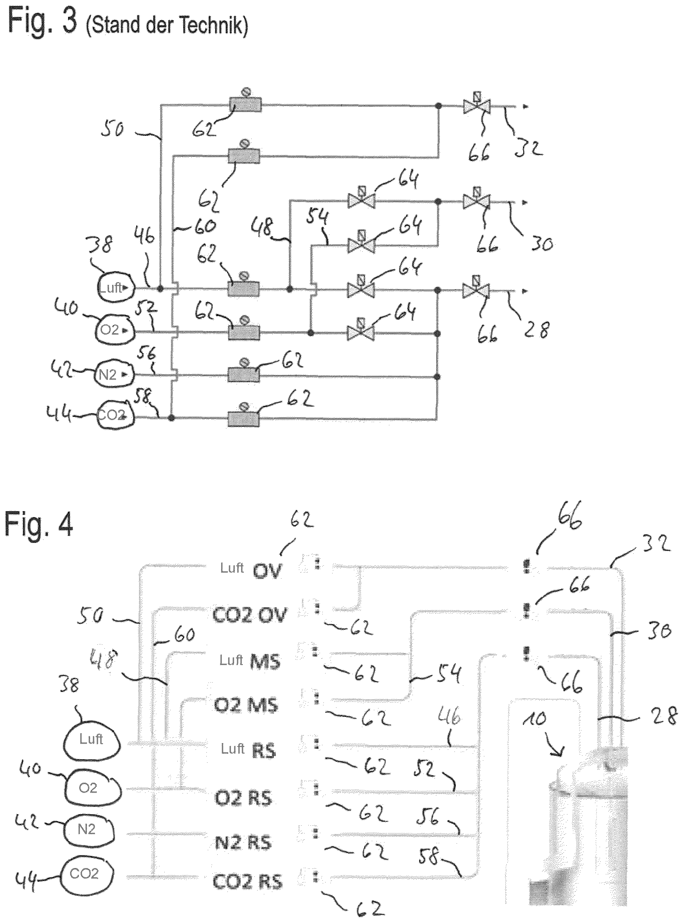

In Figur 3 ist ein Beispiel für ein Schaltbild einer herkömmlichen Gaszufuhrstation mit vier verschiedenen Gasquellen 38, 40, 42, 44 (Luft, Sauerstoff, Stickstoff und Kohlenstoffdioxid) gezeigt.

Eine Luftzufuhrleitung 46 führt von der Luftquelle 38 zur ersten Gasauslassleitung 28, die in den Ringsparger 22 mündet. Ein erster Abzweig 48 führt von der Luftzufuhrleitung 46 zur zweiten Gasauslassleitung 30, die in den Microsparger 24 mündet. Ein zweiter Abzweig 50 führt von der Luftzufuhrleitung 46 zur dritten Gasauslassleitung 32, die in den Overlay-Gasauslass mündet.

Eine Sauerstoffzufuhrleitung 52 führt von der Sauerstoffquelle 40 zur ersten Gasauslassleitung 28, die im Ringsparger 22 mündet. Ein dritter Abzweig 54 führt von der Sauerstoffzufuhrleitung 52 zur zweiten Gasauslassleitung 30, die im Microsparger 24 mündet.

Eine Stickstoffzufuhrleitung 56 führt von der Stickstoffquelle 42 zur ersten Gasauslassleitung 28, die im Ringsparger 22 mündet.

Eine Kohlenstoffdioxidzufuhrleitung 58 führt von der Kohlenstoffdioxidquelle 44 zur ersten Gasauslassleitung 28, die im Ringsparger 22 mündet. Ein vierter Abzweig 60 führt von der Kohlenstoffdioxidzufuhrleitung 58 zur dritten Gasauslassleitung 32, die in den Overlay-Gasauslass mündet.

Es sind insgesamt sechs mit einer Steuerung verbundene Massendurchflussregler 62 vorgesehen, die an folgenden Stellen angeordnet sind: 1) in der Luftzufuhrleitung 46 zwischen dem ersten Abzweig 48 und dem zweiten Abzweig 50; 2) in der Sauerstoffzufuhrleitung 52 vor dem dritten Abzweig 54; 3) in der Stickstoffzufuhrleitung 56; 4) in der Kohlenstoffdioxidzufuhrleitung 58 hinter dem vierten Abzweig; 5) im zweiten Abzweig 50; 6) im vierten Abzweig 60.

Des Weiteren sind gemäß dem Schaltbild der Figur 3 vier Schaltventile 64 an folgenden Stellen angeordnet: 1) in der Luftzufuhrleitung 46 hinter dem Massendurchflussregler 62 und dem ersten Abzweig 48; 2) in der Sauerstoffzufuhrleitung 52 hinter dem Massendurchflussregler 62 und dem dritten Abzweig 54; 3) im ersten Abzweig 48; 4) im dritten Abzweig 54. Die Schaltventile 64 werden je nach Bedarf wechselseitig so geschaltet, dass das jeweilige Gas entweder zum Ringsparger 22 oder zum Microsparger 24 geleitet wird.

Ferner kann jede der drei Gasauslassleitungen 28, 30, 32 jeweils durch ein Sperrventil 66, typischerweise ein Magnetventil, gesperrt und freigegeben werden.

Um z.B. eine Zufuhr von Sauerstoff in das im Behälter 12 des Bioreaktorsystems 10 aufgenommene flüssige biologische Medium über den Microsparger 24 zu erreichen, muss ein geeigneter Durchfluss am Massendurchflussregler 62 in der Sauerstoffzufuhrleitung 52 eingestellt werden, und es muss sichergestellt sein, dass das hinter dem dritten Abzweig 54 angeordnete Schaltventil 64 geschlossen ist, während das Schaltventil 64 im dritten Abzweig 54 und das Sperrventil 66 in der zweiten Gasauslassleitung 30 geöffnet sind.

Figur 4 zeigt beispielhaft ein Schaltbild für die Gaszufuhr eines erfindungsgemäßen Bioreaktorsystems 10. Der Verlauf der Leitungen 28, 30, 32, 46, 52, 56, 58 und der Abzweige 48, 50, 54 und 60 ist grundsätzlich der gleiche wie bei der zuvor beschriebenen Gaszufuhrstation, die als Modul des Bioreaktorsystems 10 ausgebildet sein kann. Es sind auch hier Sperrventile 66 zum Sperren und Freigeben der drei Gasauslassleitungen 28, 30, 32 vorgesehen, aber keine Schaltventile 64. Dafür kommen hier insgesamt acht mit einer Steuerung verbundene Massendurchflussregler 62 zum Einsatz, die in allen vier Gaszufuhrleitungen 46, 52, 56, 58 von den Gasquellen 38, 40, 42, 44 und in allen vier Abzweigen 48, 50, 54, 60 angeordnet sind. Das bedeutet, dass in jeder Gasstrecke ein Massendurchflussregler 62 vorgesehen ist.

Mit dieser Konfiguration ist es möglich, die Zufuhr aller Gase zum Ringsparger 22 sowie die Zufuhr von Luft und Sauerstoff zum Microsparger 24 und zum Overlay-Gasauslass individuell zu steuern. Insbesondere kann dank der Massendurchflussregler 62 für jedes Gas individuell eingestellt werden, ob und mit welchem Massendurchfluss es der jeweiligen Gasauslassleitung 28, 30, 32 zugeführt wird. Insbesondere ist es möglich, ein Gas gleichzeitig mehreren Gasauslassleitungen 28, 30, 32 zuzuführen. Beispielsweise kann Sauerstoff gleichzeitig dem Ringsparger 22 und dem Microsparger 24 mit unterschiedlichem Massendurchfluss zugeführt werden.

Selbstverständlich ist es auch möglich, mehr oder weniger und/oder andere Gasquellen vorzusehen. Ebenso ist es möglich, mehr oder weniger Gasauslassleitungen vorzusehen, die in Sparger oder direkt in das flüssige biologische Medium münden bzw. für eine Overlay-Begasung vorgesehen sind. Wichtig ist, dass für jede vorgesehene Gaszufuhr zu einer Gasauslassleitung ein eigener Massendurchflussregler 62 vorgesehen ist.

In einer Konfiguration, bei der, wie beispielhaft anhand der Figur 4 beschrieben, für jede Gaszufuhrleitung 46, 52, 56, 58 von einer Gasquelle 38, 40, 42, 44 zu einer Gasauslassleitung 28, 30, 32 und für jeden Abzweig 48, 50, 54, 60, der ebenso eine Verbindung zwischen einer Gasquelle 38, 40, 42, 44 und einer Gasauslassleitung 28, 30, 32 herstellt, ein eigener Massendurchflussregler 62 vorgesehen ist, oder in einer herkömmlichen Konfiguration, wie sie zuvor beispielhaft anhand der Figur 3 beschrieben wurde, kann eine besondere Steuerung verwendet werden, die nachfolgend beschrieben wird.

Ein (in den Figuren nicht separat dargestelltes) Mess- und Regelungssystem, nachfolgend der Einfachheit halber als "Steuerung" (Controller) bezeichnet, das hier einen Führungsregler mit wenigstens einem zugeordneten Sensor und mehreren Folgereglern mit grundsätzlich gleichzeitig ansteuerbaren Stellgliedern (hier insbesondere der Rührer 14 und die Massendurchflussregler 62) umfasst. Den Folgereglern können weitere Sensoren zugeordnet sein.

Der wenigstens eine Sensor, der dem Führungsregler zugeordnet ist, ist typischerweise ein Sensor zur Messung des Gelöstsauerstoffs (DO-Sensor). Auf andere mögliche Sensoren wird später noch im Detail eingegangen.

Die Steuerung ist so eingerichtet, dass für die Durchführung eines Bioprozesses (oder Bioprozessschritts) vom Anwender ein konstant zu haltender Sollwert für eine während des Prozesses überwachte Regelgröße eingegeben werden kann. Im hier beschriebenen Beispiel kann der Anwender also einen Sollwert für den Gelöstsauerstoff eingeben. Die Steuerung verfügt über einen DO-Sensor zur kontinuierlichen Überwachung (wiederholte Durchführung von Messungen) dieses Parameters und über einen Regler für diese Regelgröße (DO-Regler), der ein von der Abweichung des mittels des Sensors bestimmten Regelgrößen-Istwerts von dem vom Anwender eingestellten Regelgrößen-Sollwert abhängiges Ausgangssignal erzeugt.

Der DO-Regler arbeitet als Führungsregler, d.h. er wirkt je nach Konfiguration auf einen oder mehrere Folgeregler für die Steuerung von Stellgliedern, die den DO-Wert im Prozess gezielt beeinflussen können. Bei Abweichung vom Sollwert gibt der Führungsregler ein Ausgangssignal (Führungsgröße) auf die Folgeregler, das relativ zum Regelbereich ist (0% ... 100%). Die Folgeregler steuern entsprechend diesem Ausgangssignal ihre Stellglieder (hier insbesondere den Rührer 14 für die Durchmischung und die Massendurchflussregler 62 für die Begasung) an. Genauer gesagt ist für jeden Folgeregler ein Steuerprofil hinterlegt, das ihm in Abhängigkeit vom Ausgangssignal des Führungsreglers sein Regelverhalten vorgibt.

Im hier betrachteten Beispiel lassen sich jedem Folgeregler mehrere (z.B. fünf) Sollwerte zuweisen, abhängig vom Ausgangssignal des Führungsreglers. Beispielsweise kann für die Ausgangswerte 0%, 10%, 20%, 30%, 40% und 50% des Führungsreglers jeweils ein Sollwert für die Stellglieder der Folgeregler eingegeben werden. In der Regel gibt der Anwender diese Sollwerte selbst vor. Die Sollwerte können aber auch vom Hersteller vorgegeben und in einem Speicher der Steuerung hinterlegt sein. Die Sollwerte können auch in einer externen Datenbank hinterlegt sein, auf die die Steuerung Zugriff hat. Die Sollwerte für die Folgeregler sind auf die konkrete Bioreaktor-Konfiguration abgestimmt und basieren auf Erfahrungswerten und/oder einem mathematischen Analysemodell, das durch eine umfassende verfahrenstechnische Charakterisierung erstellt worden ist.

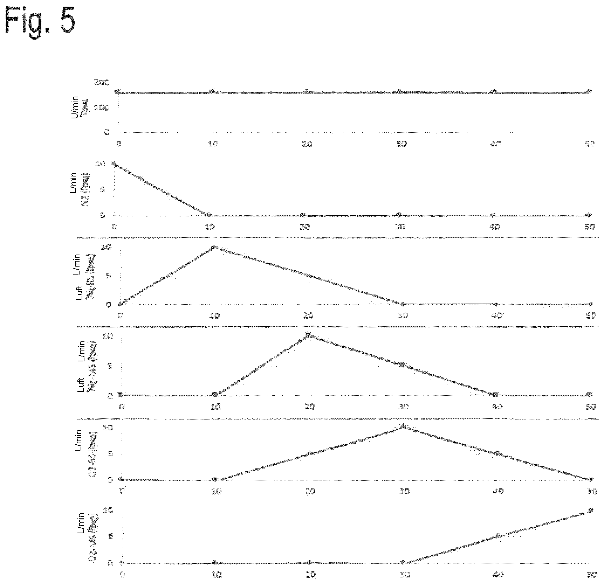

Eine Besonderheit der Steuerung liegt darin, dass die Sollwerte für die Folgeregler nicht in abstrakten Ausgabewerten relativ zu deren Regelbereichen (0% ... 100%) eingegeben werden müssen. Vielmehr können die Sollwerte in der physikalischen Einheit der jeweiligen Stellgröße eingegeben werden. Im hier betrachteten Beispiel kann der Anwender also für die verschiedenen Ausgangswerte des DO-Reglers jeweils eine konkrete Drehzahl (U/min) für den Rührer 14 und konkrete Begasungsraten (Umin) für die Massendurchflussregler 62 eingeben. Die so festgelegten Sollwerte lassen sich als Polygonzüge über dem Ausgangssignal des Führungsreglers grafisch darstellen, wie beispielhaft in Figur 5 gezeigt, wobei die x-Achse den Ausgang des Führungsreglers und die y-Achsen die zugehörigen Stellgrößen der Stellglieder in der physikalischen Einheit der jeweiligen Stellgröße angeben.

Als Sicherheitsfunktion der Steuerung kann vorgesehen sein, dass bei Eingabe von Sollwerten für den Rührer 14 und die Massendurchflussregler 62, die zu einem zu hohen Gaseintrag bzw. einer zu hohen Rührdrehzahl und einem dadurch bedingten sogenannten "Impeller Flooding" (Überflutungsverhalten) führen würden, eine Warnung ausgegeben wird, oder dass die Eingabe solcher Werte erst gar nicht akzeptiert wird. Auf die gleiche Weise kann auf Eingaben reagiert werden, die unnötig hohe Leistungseinträge ohne Begasung zur Folge hätten.

Unter Bezugnahme auf das hier betrachtete Beispiel wird im Betrieb, d.h. während des laufenden Prozesses, wiederholt der vom DO-Sensor gemessene DO-Wert mit dem vom Anwender vorgegebenen Sollwert verglichen, und der DO-Regler gibt entsprechend der festgestellten Abweichung ein Ausgangssignal relativ zu seinem Regelbereich aus. Die Folgeregler regeln automatisch den Rührer 14 und die Massendurchflussregler 62 auf die zuvor für diesen Ausgangswert des DO-Reglers eingestellten Sollwerte, sodass der DO-Sollwert gehalten wird.

Bei der in Figur 5 beispielhaft gezeigten grafischen Darstellung mehrerer Polygonzüge erfolgt die Regelung auf einen vom Anwender vorgegebenen Sollwert für die Regelgröße durch entsprechende Ansteuerung des Rührers 14 und der Massendurchflussregler 62 so, dass die Rührerdrehzahl (oberstes Diagramm) über den gesamten interessierenden Regelbereich des Führungsreglers (hier 0% bis 50%) auf 160 U/min gehalten wird. Die Stickstoffzugabe über den Ringsparger 22 (zweites Diagramm von oben) beträgt bei einem Ausgangswert von 0% des Führungsreglers 10 L/min und ab einem Ausgangswert von 10% des Führungsreglers 0, d.h. es erfolgt auch bei größeren Abweichungen keine Stickstoffzugabe mehr. Luft wird sowohl über den Ringsparger 22 (drittes Diagramm von oben) als auch über den Microsparger 24 (viertes Diagramm von oben) zugeführt. Die jeweiligen Sollwerte sind ähnlich, aber bezüglich der Ausgangswerte des Führungsreglers versetzt. Für die Zufuhr von Kohlenstoffdioxid über den Ringsparger 22 (fünftes Diagramm von oben) und den Microsparger 24 (sechstes Diagramm von oben) sind jeweils unterschiedliche Polygonzüge vorgesehen.

Gemäß einer anderen Ausführungsform der Steuerung werden mit den Ausgabewerten des Führungsreglers keine Sollwerte für die Stellgrößen der Stellglieder verknüpft, sondern wenigstens ein Sollwert für einen Parameter, der unmittelbar auf den Prozess bezogen ist und der mit den Stellgliedern gezielt beeinflusst werden kann. Mit anderen Worten: Der Anwender ordnet den verschiedenen Ausgabewerten des Führungsreglers keine Sollwerte für den Rührer 14 und die Massendurchflussregler 62 zu, sondern gibt einfach jeweils einen Sollwert für einen durch diese Stellglieder steuerbaren Parameter ein, wie etwa eine gewünschte Zellwachstumsrate, Zelldichte, Sauerstofftransferrate (OTR), Sauerstoffaufnahmerate (OUR), Blasengröße, Blasenanzahl, Mischzeit oder einen gewünschten volumenbezogenen Stoffübergangskoeffizienten (k_{L}a-Wert).

Mit solchen Parametern ist der Anwender in der Regel vertraut. Der k_{L}a-Wert ist in einem biotechnologischen Prozess etwa von großer Bedeutung, da er eine Aussage darüber trifft, wie gut Mikroorganismen im Behälter 12 des Bioreaktorsystems 10 mit Gasen versorgt werden können. Bei der Kultivierung aerober Mikroorganismen ist es wichtig, einen hohen k_{L}a-Wert für Sauerstoff zu erreichen, während bei autotroph wachsenden Mikroorganismen auch die k_{L}a-Werte für Wasserstoff und Kohlenstoffdioxid eine wichtige Rolle spielen.

Wenn der Anwender einen Prozess im Labormaßstab mit einem kleinen Bioreaktor entwickelt hat, kennt er die optimale Werte für einen oder mehrere solcher Parameter. Die zum Erreichen des gewünschten Parameterwerts notwendigen Einstellungen der Stellglieder, insbesondere des Rührers und der Massendurchflussregler, lassen sich aber nicht 1:1 auf einen großvolumigen Prozess in einem großen Bioreaktor mit anderen Stellgliedern, insbesondere einem größerem Rührer 14 und Spargern 22, 24, übertragen. Das bedeutet, dass für das Erreichen eines gewünschten Parameterwerts andere Sollwerte für die Stellglieder vorgegeben werden müssen, die der Anwender aber erst in Erfahrung bringen muss.

Diese Arbeit nimmt ihm die Steuerung ab. Der Anwender gibt nur den gewünschten Parameterwert als Sollwert ein (z.B. einen k_{L}a-Sollwert), den er aus dem von ihm im Labormaßstab entwickelten Prozess kennt, und die Steuerung weist diesem Parametersollwert ein Steuerprofil zu, mit dem der ParameterSollwert mit dem großen Bioreaktor im Produktionsmaßstab erreicht wird. Das Steuerprofil umfasst Sollwerte für die Folgeregler-Stellglieder, wie sie weiter oben beschrieben wurden. Die Zuordnung dieser Folgeregler-Sollwerte zu einem gewünschten Parameterwert basiert wiederum auf einem Analysemodell, das durch eine umfassende verfahrenstechnische Charakterisierung erstellt worden ist. Beispielsweise sind für verschiedene k_{L}a-Werte jeweils Sollwerte für den Rührer 14 und die Massendurchflussregler 62 hinterlegt, die auf die vorliegende Bioreaktor-Konfiguration abgestimmt sind. Grundlage hierfür ist, dass der k_{L}a-Wert u.a. von der Rührerdrehzahl und der Kontaktfläche zur Luft (Blasengröße) abhängt.

Gemäß einer Weiterbildung der Steuerung ist es auch möglich, einen anderen Parameter als den von einem DO-Sensor gemessenen DO-Wert als Regelgröße für die Regelung des Prozesses zu verwenden. Als Regelgröße kommt insbesondere einer der folgenden Parameter infrage, die während des Prozesses direkt mit entsprechenden Sensoren überwacht werden können: CO₂-Gehalt, Zellzahl, Zelldichte, Lebendzellvolumen, Trübung, Glukosegehalt, pH-Wert, Kohlenstoffdioxidpartialdruck (pCO₂), Sauerstoffpartialdruck (pO₂).

Darüber hinaus ist es auch möglich, Parameter als Regelgröße für die Regelung des Prozesses heranzuziehen, die nicht direkt mit einem Sensor messbar sind, sondern erst aus Messwerten von einem oder mehreren Sensoren berechnet oder mithilfe eines zuvor erstellten Modells bestimmt werden können. Zu diesen Parametern zählen u.a. die Sauerstofftransferrate (OTR), die Sauerstoffaufnahmerate (OUR), die Zellwachstumsrate, die Glukoseverbrauchsrate, die Kohlenstoffdioxidbildungsrate (CER) und -austragungsrate, der respiratorische Quotient (RQ-Wert), der Biomasseertrag (biomass yield), die Glukoseausbeute und allgemein die Ausbeute anderer Kohlenstoff- oder Stickstoffquellen. Die Hardware (Sensor bzw. Sensoren) und die Software (Algorithmen, Zuweisungstabellen etc.), die zur Bestimmung solcher Parameter während des Prozesses benötigt werden, können gemeinsam verkürzt als "Softsensoren" bezeichnet werden.

Es ist auch möglich, alternativ oder zusätzlich die Messwerte eines Abgassensors (off-gas sensor) zu berücksichtigen, der z.B. den Sauerstoff- oder Kohlenstoffdioxidgehalt des aus dem Behälter 12 des Bioreaktorsystems 10 abgeführten Abgases misst.

Es können auch spektroskopische Analysegeräte (z.B. IR-, UV/VIS-, Raman-Spektrometer etc.) als "Sensoren" zur Bestimmung eines Regelgröße-Parameters herangezogen werden. Ebenso können Einrichtungen zur Durchführung von Messungen in Bezug auf Stoffe, die die Zellen im Bioreaktorbehälter verstoffwechseln oder als Induktoren für eine Genexpression dienen (z.B. Laktat, Methanol/Ethanol etc.) als Sensoren Verwendung finden.

Die Steuerung kann zudem so eingerichtet sein, dass der Anwender je nach Anwendung der Parameter entweder ein heterogenes oder ein homogenes Blasenbild einstellen kann. Ein heterogenes Blasenbild sorgt für ein effektiveres Mischen, ein homogenes Blasenbild für einen verbesserten Gastransfer. Die Begasungsrate (Massendurchfluss) und die Rührerdrehzahl werden von der Steuerung automatisch so abgestimmt, dass sich das gewünschte Blasenbild einstellt.

Die Regelung des Prozesses auf eine andere Regelgröße als den DO-Wert erfolgt grundsätzlich auf die gleiche Weise, wie weiter oben beschrieben, d.h. einem Sollwert für eine solche Regelgröße können Sollwerte für die Stellglieder von Folgereglern in der physikalischen Einheit der jeweiligen Stellgröße oder Sollwerte für einen Parameter eingegeben werden, der unmittelbar auf den Prozess bezogen ist. In letzterem Fall weist die Steuerung automatisch passende Sollwerte für die Stellglieder zu.

Je nach Regelgröße können Folgeregler vorgesehen sein, die zusätzlich oder alternativ zum Rührer 14 und den Massendurchflussreglern 62 andere Stellglieder aufweisen, etwa eine Pumpe zum Zuführen eines Mediums (z.B. einer Nährlösung oder eines Puffers).

Die Eingabe der Sollwerte für die Regelgröße(n) und der vom Ausgabewert des Führungsreglers abhängigen Sollwerte für die Stellglieder der Folgeregler bzw. einen prozessbezogenen Parameter kann über ein Software-Layer erfolgen, das über einem herkömmlichen Controller liegt. Die Steuerung kann aber auch von vornherein speziell für die Verarbeitung solcher eingegebenen Sollwerte ausgebildet sein.

Die beispielhaft beschriebenen Konfigurationen der Begasungsanlage und der Steuerung eignen sich grundsätzlich für alle Bioreaktorgrößen.

### Bezuaszeichenliste

- 10: Bioreaktorsystem
- 12: Behälter
- 14: Rührer
- 16: Rührerwelle
- 18: Rührorgan
- 20: Begasungsvorrichtung
- 22: (äußerer) erster Begasungskanal; Ringsparger
- 24: (innerer) zweiter Begasungskanal; Microsparger
- 26: Gehäuse
- 28: erste Gasauslassleitung (Ringsparger)
- 30: zweite Gasauslassleitung (Microsparger)
- 32: dritte Gasauslassleitung (Overlay)
- 34: Gasauslassöffnungen des ersten Begasungskanals
- 36: Gasauslassöffnungen des zweiten Begasungskanals
- 38: Luftquelle
- 40: Sauerstoffquelle
- 42: Stickstoffquelle
- 44: Kohlenstoffdioxidquelle
- 46: Luftzufuhrleitung
- 48: erster Abzweig
- 50: zweiter Abzweig
- 52: Sauerstoffzufuhrleitung
- 54: dritter Abzweig
- 56: Stickstoffzufuhrleitung
- 58: Kohlenstoffdioxidzufuhrleitung
- 60: vierter Abzweig
- 62: Massendurchflussregler
- 64: Schaltventil
- 66: Magnetventil

## Patentansprüche

1. Bioreaktorsystem (10) zur Durchführung eines biologischen Prozesses, mit
einem Behälter (12) zur Aufnahme eines flüssigen biologischen Mediums, und einer Begasungsanlage zur kontrollierten Zufuhr verschiedener Gase von Gasquellen (38, 40, 42, 44) in den Behälter (12),
wobei die Begasungsanlage mehrere Gasauslassleitungen (28, 30, 32) aufweist, die in eine oder mehrere Begasungsvorrichtungen (20) und/oder in einen Overlay-Gasauslass im Inneren des Behälters (12) münden,
wobei jede Gasauslassleitung (28, 30, 32) über jeweils eine Gaszufuhrleitung (46, 52, 56, 58) oder jeweils einen Abzweig (48, 50, 54, 60) von einer Gaszufuhrleitung (46, 52, 56, 58) mit mehreren Gasquellen (38, 40, 42, 44) verbunden ist, und
wobei in jeder Gaszufuhrleitung (46, 52, 56, 58) und in jedem Abzweig (48, 50, 54, 60) ein mit einer Steuerung verbundener Massendurchflussregler (62) angeordnet ist.

2. Bioreaktorsystem (10) nach Anspruch 1, **dadurch gekennzeichnet, dass** eine erste Gasauslassleitung (28), die in einen Ringsparger (22) mündet, mit einer Luftquelle (38) und/oder einer Sauerstoffquelle (40) und/oder einer Stickstoffquelle (42) und/oder einer Kohlenstoffdioxidquelle (44) verbunden ist.

3. Bioreaktorsystem (10) nach Anspruch 1 oder 2, **dadurch gekennzeichnet, dass** eine zweite Gasauslassleitung (30), die in einen Microsparger (24) mündet, mit einer Luftquelle (38) und/oder einer Sauerstoffquelle (40) verbunden ist.

4. Bioreaktorsystem (10) nach einem der vorhergehenden Ansprüche, **dadurch gekennzeichnet, dass** eine dritte Gasauslassleitung (32), die in einen Overlay-Gasauslass mündet, mit einer Luftquelle (38) und/oder einer Kohlenstoffdioxidquelle (44) verbunden ist.

5. Bioreaktorsystem (10) zur Durchführung eines biologischen Prozesses, insbesondere nach einem der vorhergehenden Ansprüche, **gekennzeichnet durch** eine Steuerung, die einen Führungsregler für eine Regelgröße, wenigstens einen dem Führungsregler zugeordneten Sensor und einen oder mehrere Folgeregler mit Stellgliedern (14, 62) umfasst, deren Stellgrößen die Regelgröße gezielt beeinflussen,
wobei die Steuerung so eingerichtet ist, dass
- dem Führungsregler ein Sollwert für die Regelgröße vorgegeben werden kann,
- der wenigstens eine Sensor zur wiederholten Bestimmung eines Istwerts der Regelgröße verwendet wird, und
- der Führungsregler ein von der Abweichung des Regelgrößen-Istwerts vom Regelgrößen-Sollwert abhängiges Ausgangssignal auf die Folgeregler gibt, wobei den Folgereglern Steuerprofile zugewiesen sind, die vom Ausgangssignal des Führungsreglers abhängig sind, und
wobei die Steuerprofile Sollwerte für die Stellglieder (14, 62) der Folgeregler beinhalten und die Steuerung so eingerichtet ist, dass die Sollwerte für die Stellglieder (14, 62) in der physikalischen Einheit von deren Stellgrößen eingegeben werden können, oder
wobei die Steuerprofile Sollwerte für einen unmittelbar auf den biologischen Prozess bezogenen, durch die Stellglieder (14, 62) gezielt beeinflussbaren Parameter beinhalten und die Steuerung so eingerichtet ist, dass sie den Parameter-Sollwerten automatisch Sollwerte für die Stellglieder (14, 62) der Folgeregler zuweist.

6. Bioreaktorsystem (10) nach Anspruch 5, **dadurch gekennzeichnet, dass** der Parameter einer der Folgenden ist: Zellwachstumsrate, Zelldichte, Sauerstofftransferrate, Sauerstoffaufnahmerate, Blasengröße, Blasenanzahl, Mischzeit, volumenbezogener Stoffübergangskoeffizient.

7. Bioreaktorsystem (10) nach Anspruch 5 oder 6, **dadurch gekennzeichnet, dass** die Regelgröße ein Parameter ist, der direkt mit dem wenigstens einen Sensor messbar ist, insbesondere wenigstens einer der Folgenden: Gelöstsauerstoff, Kohlenstoffdioxidgehalt, Zellzahl, Zelldichte, Lebendzellvolumen, Trübung, Glukosegehalt, pH-Wert, Kohlenstoffdioxidpartialdruck, Sauerstoffpartialdruck.

8. Bioreaktorsystem (10) nach Anspruch 5 oder 6, **dadurch gekennzeichnet, dass** die Regelgröße ein Parameter ist, der nicht direkt mit dem wenigstens einen Sensor messbar, aber mithilfe des wenigstens einen Sensors bestimmbar ist, insbesondere wenigstens einer der Folgenden: Sauerstofftransferrate, Sauerstoffaufnahmerate, Zellwachstumsrate, Glukoseverbrauchsrate, Kohlenstoffdioxidbildungsrate, Kohlenstoffdioxidaustragungsrate, respiratorischer Quotient, Biomasseertrag, Glukoseausbeute, Ausbeute einer Kohlenstoff- oder Stickstoffquelle.

9. Bioreaktorsystem (10) nach einem der Ansprüche 5 bis 8, **dadurch gekennzeichnet, dass** die Stellglieder einen in einem Behälter (12) des Bioreaktorsystems angeordneten Rührer (14) und mehrere Massendurchflussregler (62) einer Begasungsanlage umfassen und dass die Steuerung so eingerichtet ist, dass als Sollwerte für den Rührer (14) Werte in der Einheit U/min und als Sollwerte für die Massendurchflussregler Werte in der Einheit Umin eingegeben werden können.

10. Bioreaktorsystem (10) nach einem der Ansprüche 5 bis 9, **dadurch gekennzeichnet, dass** die Steuerprofile in der Steuerung hinterlegt sind.

11. Bioreaktorsystem (10) nach einem der Ansprüche 5 bis 9, **dadurch gekennzeichnet, dass** die Steuerprofile in einer externen Datenbank hinterlegt sind, auf die die Steuerung Zugriff hat.

12. Bioreaktorsystem (10) nach einem der Ansprüche 5 bis 11, **dadurch gekennzeichnet, dass** die Steuerprofile auf empirischen Werten oder auf einem mathematischen Modell basieren.

13. Bioreaktorsystem (10) nach einem der Ansprüche 5 bis 12, **dadurch gekennzeichnet, dass** die Steuerung die Eingaben der Sollwerte für die Folgeregler, insbesondere den Rührer (14) und die Massendurchflussregler (62), dahingehend überwacht, dass keine Sollwertkombinationen eingegeben werden, die zu einem nicht-ordnungsgemäßen Betrieb des Bioreaktorsystems (10) führen würden.
